# EUROPEAN PATENT SPECIFICATION

(11) **EP 1 361 906 B1**
(45) Date of publication and mention of the grant of the patent: **11.04.2007**
(21) Application number: 02714923.6
(22) Date of filing: 14.02.2002
(51) Int. Cl.: A61L 31/04, A61L 31/14, A61L 31/06, A61L 15/26, A61L 15/42, A61L 24/04, A61L 24/00, A61L 26/00, A61L 27/18, A61L 27/38, A61L 27/56

(54) **BIOCOMPATIBLE FLEECE FOR HEMOSTASIS AND TISSUE ENGINEERING**
BIOKOMPATIBLES VLIES FÜR HÄMOSTASE UND GEWEBEAUFBAU
MOLLETON BIOCOMPATIBLE POUR HEMOSTASE ET TISSU OBTENU PAR GENIE TISSULAIRE

(30) Priority: 14.02.2001 US 268559 P
(43) Date of publication of application: 19.11.2003
(73) Proprietor: GENZYME CORPORATION, Cambridge, Massachusetts 02139 (US)
(72) Inventor: AVILA, Luis, A., Arlington, MA 02474-2124 (US); PHILBROOK, C., Michael, Boston, MA 02115 (US); BASSETT, Michael, J., Rumford, RI 02916 (US); DOHERTY, Edward, Mansfield, MA 02048 (US); TRAVERSE, John, F., Milton, MA 02186 (US); JARRETT, Peter, K., Sudbury, MA 01776 (US); HUIBREGTSE, Barbara, A., Shrewsbury, MA 01545 (US); BROWN, Liesbeth, M., E., West Newton, MA 02465 (US); MESSIER, Kenneth, A., Griswold, CT 06351 (US); KRAMER, Hildegard M., Westport, CT 06680 (US)
(74) Representative: Hermann, Bettina Celia
(86) International application number: PCT/US2002/004638
(87) International publication number: WO 2002/064182

(56) References cited:
- EP-A- 0 747 068
- WO-A-01/02033
- WO-A-01/06973
- WO-A-98/22154
- US-A- 5 410 016
- US-A- 5 800 537
- US-A- 5 900 245

## Description

### BACKGROUND OF THE INVENTION

### 1. Field of the Invention

This invention is generally in the field of polymeric materials useful for medical applications and tissue engineering.

### 2. Description of the Related Art

Porous materials have multiple uses in medicine and biotechnology. In general, the materials used are either microporous, having pores smaller than about one micron; or macroporous, having pores in the range of microns to millimeters. The microporous materials are generally gels, or in some cases foams or microporous membranes. Because of the pore size, cells cannot penetrate the microporous matrix. This is an advantage for some applications, such as filtration and the formation of barriers on tissue, but not in cell cultivation or immobilization.

Macroporous materials are typically coarser open-cell forms, such as foamed gelatin (e.g., "GelFoam"; Abbott), or are made by crosslinked or nonwoven aggregates of filaments (gauze, for example). Such techniques have been used to make macroporous structures of (from) biodegradable materials such as lactic acid, glycolic acid, and copolymers. Macroporous media allow cell ingress or attachment, but usually lack the hydrophilicity and biocompatibility of a gel.

In one medical application, there has been substantial interest in developing a more facile method of delivering cells to repair localized tissue damage. In the specific case of defects of the articular cartilage in the knee, such defects may progress to osteoarthritis and require total knee replacement. Autologous Chondrocyte Implantation (ACI) has been used to treat people with deep cartilage defects in the knee. ACI involves obtaining healthy chondrocytes from an uninvolved area of the injured knee during arthroscopy. The chondrocytes are then isolated and cultured. The cultured chondrocytes are then injected into the area of the defect. The defect is covered with a sutured periosteal flap taken from the proximal medial tibia. The procedure is very time consuming and requires the periosteal flap to be sutured sufficiently to seal the chondrocytes into the area of the defect. See M. Brittberg, et al., New England J. of Med. 331, 889 (1994). Improvements have been disclosed to cartilage repair procedures such as by using chondrocyte cells retained to an absorbable support matrix, B. Gianetti etal., WO 00/09179; by using low density seeded chondrocytes, T. Gagne et al., WO 98/55594; by using a hydrogel support containing tissue precursor cells, U.S. Patent 6,027,744 to C. Vacanti et al.; chondrocyte cells seeded in a collagen matrix, U.S. Patent 4,846,835 of D. Grande; chondrocyte cells seeded in a fibrous, polymeric matrix, U.S. Patent 5,041,138 to J. Vacanti et al.; and chondrocyte cells seeded on various other supports, U.S. Patents 5,326,357; 6,206,931; 5,837,278; 5,709,854; and PCT Application WO 01/08610. There is, however, a need to improve cartilage repair procedures to increase the ease of application and effectiveness in repairing tissue damage.

It is therefore an object of the present invention to provide materials with properties that combine macroporosity and gel-like microporosity.

It is a further object of the present invention to provide uses for these materials in medicine and biotechnology.

It is a further object of the present invention to provide uses for these materials to facilitate the repair of wounds and defects of the body, particularly defects of the articular cartilage in the knee.

### SUMMARY OF THE INVENTION

It has been discovered that crosslinkable polymeric materials, normally used to form gels, can be used to form macroporous materials having both gel properties and macroporosity. The process is simple and reproducible, and allows control of the porosity and swelling properties of the resulting fleece. In its simplest embodiment, gels are formed by dissolving a crosslinkable polymer in water (without crosslinking it); freezing the aqueous solution; lyophilizing the solution to form a dry, porous fleece; and crosslinking the polymers in the fleece state. The fleece is stable for long periods at room temperature, especially if kept dry, but rehydrates rapidly in the presence of water or biological fluids, which optionally may contain living cells. Several variations on the procedure are possible, including crosslinking in the frozen state; making a fleece with multiple layers by adding successive layers, optionally containing different materials, to previously frozen layers before lyophilization; incorporation of bioactive materials, such as drugs, growth factors and hemostatic agents and cells; and provision of varying degrees of biodegradability.

Objects and features of the present invention will become apparent from the following detailed description.

The invention is in accordance with the claims.

### DETAILED DESCRIPTION OF THE PRESENTLY PREFERRED EMBODIMENTS

### MATERIALS FORMING THE FLEECE

As used herein, a fleece is a porous material which swells in the presence of water, and which has both macroporosity, and, when hydrated, microporosity. The fleece is a crosslinked material having the properties of biocompatibility, biodegradability, and the ability to absorb aqueous solutions. The fleece is formed by crosslinking crosslinkable polymeric molecules (macromers). In a preferred embodiment, the material is to be applied to tissue, or used to form a support for tissue repair. The compositions may further contain other agents, including biologically-active materials and living cells.

### Crosslinkable Materials

As used herein, "crosslink" is defined generically, to refer to the joining of smaller entities to form a structure by any physical or chemical means. Unless stated otherwise, the term "polymerize" is a functional equivalent of "crosslink".

In U.S. Patent No. 5,410,016 to Hubbell et al., application of biodegradable macromers to tissue, followed by photopolymerization to form a crosslinked gel, is described. In addition to the photopolymerizable gels described by Hubbell et al., systems for forming drug delivery depots or barriers on surfaces include the polymers described in U.S. Patent No. 4,938,763 to Dunn, et al., U.S. Patent Nos. 5,100,992 and 4,826,945 to Cohn et al, U.S. Patent Nos. 4,741,872 and 5,160,745 to De Luca et al, US 5,527,864 to Suggs et al, U.S. Patent No. 4,511,478 to Nowinski et al, and U.S. patent 4,888,413 to Domb. These materials, which covalently crosslink by free-radical-initiated polymerization, are preferred materials. However, materials which crosslink by other mechanisms, such as by the reaction of polyisocyanates, or other crosslinking nucleophilic groups such as succinimidates, with polyamines, or which comprise low-molecular weight reactive monomers, are also potentially suitable if they are biocompatible and non-toxic. The macro-monomers ("macromers") which are crosslinkable to form hydrogels may comprise a block copolymer. The macromers can be quickly crosslinked from aqueous solutions. The macromers may advantageously be capable of crosslinking by thermoreversible gelation, and may be crosslinked from a solution state, from a gel state, or from a solid state. In particular, materials, which can be crosslinked in a frozen state or a lyophilized state, are preferred.

Preferably, the macromers are soluble in a solvent and crosslinked from a solution state. In one aspect, the crosslinkable macromer is soluble in a solvent to a sufficient concentration to form the desired fleece. The solvent is preferably at least about 50% water, more preferably 90% to 100%. However, the solvent may contain non-aqueous liquids to any extent, subject to the limitation that the solvent can be frozen and subsequently removed by lyophilization. For example, up to about 90% of water-miscible liquids, including for example lower alcohols, acetone, DMF, DMSO, pyrrolidone, and other water miscible liquids of low toxicity, can be included in the solution to be frozen. Non-water miscible liquids can also be used as components of the solvent, provided that the resulting lyophilized product has appropriate properties. It is preferable to minimize the use of non-volatile liquids for processing. The aqueous solution may also contain buffers and other materials, such as (without limitation) initiators for polymerization, electron transfer reagents, biologically active materials, and colloids and nutrients for cell culture.

### Crosslinkable Groups

The monomers or macromers preferably include crosslinkable groups that are capable of forming covalent bonds while in a frozen state or a lyophilized state. These crosslinkable groups permit crosslinking of the macromers to form a gel. The macromers may optionally also gel by thermally-reversible or by ionic interactions of the macromers. Chemically or ionically crosslinkable groups known in the art may be provided in the macromers to provide crosslinking potential. The crosslinkable groups in one preferred embodiment are polymerizable by free radical initiation, most preferably generated by peroxygens or by visible or long wavelength ultraviolet radiation, preferably with photoinitiators. The preferred crosslinkable groups are unsaturated groups, especially ethylenic groups, including without limitation vinyl groups, allyl groups, cinnamates, acrylates, diacrylates, oligoacrylates, methacrylates, dimethacrylates, oligomethacrylates, (meth)acrylamides, acrylic esters including hydroxyethylmethacrylates, and other biologically acceptable free radical polymerizable groups. These groups can also be crosslinked by chemical or thermal means, or by any combination of chemical, thermal and photointiation means.

Other crosslinking chemistries which may be used include, for example, reaction of amines or alcohols with isocyanate or isothiocyanate, or of amines or thiols with aldehydes, activated esters, ethylenic groups, electrophilic carbon centers such as alkylhalides, epoxides, oxiranes, or cyclic imines; where either the amine or thiol, or the other reactant, or both, may be covalently attached to a macromer. Copolymers from mixtures of monomers are also contemplated. Sulfonic acid or carboxylic acid groups may also be contained in the monomers.

Preferably, at least a portion of the macromers will be crosslinkers, i.e., will have more than one crosslinkable reactive group, to permit formation of a coherent hydrogel by ensuring the crosslinking of the polymerized macromers. Up to 100% of the macromers may have more than one reactive group. Typically, in a synthesis, the percentage will be on the order of 50 to 95%, for example, 60 to 80%. The percentage may be reduced by addition of co-monomers containing only one active group. A lower limit for crosslinker concentration will depend on the properties of the particular macromer and the total macromer concentration, but will be at least about 2% of the total molar concentration of reactive groups. More preferably, the crosslinker concentration will be at least 10%, with higher concentrations, such as 30% to 90%, being optimal for maximum retardation of diffusion of many drugs. Optionally, at least part of the crosslinking function may be provided by a low-molecular weight crosslinker.

When the reactive group is a reactive group which reacts with only one other group (for example, an isocyanate), then at least some, for example at least about 1%, preferably 2% or more, more typically 5% or more, and optionally up to 100%, of the reactive molecules must contain three or more reactive groups to provide crosslinking. In some chemistries, such as epoxides reacting with primary amines, one group will be mono-reactive (in this example, epoxide) and the other will be multifunctional (in this case, amine, which can react with at least two epoxides). In such a reaction, there are several ways in which the required amount of crosslinking can be supplied, with a minimum requirement of some tri-epoxide or some dimeric primary amine. Choosing suitable mixtures is known in the art.

When a living cell or biologically active agent is to be delivered, such as a macromolecule, higher ranges of polyfunctional macromers (i.e., having more than one reactive group) are preferred. If the gel is to be biodegradable, as is preferred in most applications, then the crosslinking reactive groups in the molecule should be separated from each other by biodegradable links. Any linkage known to be biodegradable under *in vivo* conditions may be suitable, such as a degradable polymer block. The use of ethylenically unsaturated groups, crosslinked by free radical polymerization with chemical and/or photoactive initiators, is preferred as the crosslinkable group.

The macromer may also include an ionically charged moiety covalently attached to a macromer, which optionally permits gelation or ionic crosslinking of the macromer.

### Hydrophilic Regions.

The macromers have significant hydrophilic character so as to form water-absorbent gel structures. At least some of the macromers, and preferably most of the macromers, contain hydrophilic domains. A hydrophilic domain in a macromer is a hydrophilic group, block, or region of the macromer that would be water soluble if prepared as an independent molecule rather than being incorporated into the macromer. Hydrophilic groups are required for water dispersibility or solubility, and for retention of water by the gel after gelation, or upon rehydration after drying. The hydrophilic groups of the macromers are preferably made predominantly or entirely of synthetic materials. Synthetic materials of controlled composition and linkages are typically preferred over natural materials due to more consistent degradation and release properties.

Examples of useful synthetic materials include those prepared from poly(ethylene glycol) (or the synonymous poly(ethylene oxide) or polyoxyethylene), poly(propylene glycol), partially or fully hydrolyzed poly(vinyl alcohol), poly(vinylpyrrolidone), poly(ethyloxazoline), poly(ethylene oxide)-co-poly(propylene oxide) block copolymers (poloxamers and meroxapols), and poloxamines. Preferably, the water-soluble polymeric blocks are made from poly(ethylene oxide). Preferably, at least 50% of the macromers is formed of synthetic materials.

The hydrophilic groups of the macromers may also be derived from natural materials. Useful natural and modified natural materials include carboxymethyl cellulose, hydroxyalkylated celluloses such as hydroxyethyl cellulose and methylhydroxypropyl cellulose, polypeptides, polynucleotides, polysaccharides or carbohydrates such as Ficoll^{™} polysucrose, hyaluronic acid and its derivatives, dextran, heparan sulfate, chondroitin sulfate, heparin, or alginate, and proteins such as gelatin, collagen, albumin, or ovalbumin. Preferably the percentage of natural material does not exceed about 50% percent.

As used herein, a water-soluble material, such as a macromer containing a hydrophilic domain, is one that is soluble to at least 1% by weight in an aqueous solution.

### Biodegradable Regions

Biodegradable linkages or polymer or copolymer segments from molecules available in the art may be incorporated into the macromers. The biodegradable region is spontaneously hydrolyzable under *in vivo* conditions. In some embodiments, different properties, such as biodegradability and hydrophobicity or hydrophilicity, may be present within the same region of the macromer.

Useful hydrolyzable groups include polymers, oligomers and monomeric units derived from glycolide, lactide, epsilon-caprolactone, and other hydroxy acids, and other biologically degradable polymers that yield materials that are non-toxic or present as normal metabolites in the body. Preferred poly(alpha-hydroxy acids) are poly(glycolic acid), poly(DL-lactic acid) and poly(L-lactic acid). Other useful materials include poly(amino acids), polycarbonates (especially alkyl polycarbonates including poly (trimethylene carbonate), polydioxanones, poly(anhydrides), poly(orthoesters), poly(phosphazines) and poly(phosphoesters). Polylactones such as poly(epsilon-caprolactone), poly(delta-caprolactone), poly(delta-valerolactone) and poly(gamma-butyrolactone), for example, are also useful. Mixtures of these degradable linking groups may be used. The biodegradable regions may have a degree of polymerization ranging from one up to values that would yield a product that was not substantially water soluble. Thus, monomeric, dimeric, trimeric, oligomeric, and polymeric regions may be contained in the macromers.

Biodegradable regions can be constructed from polymers or monomers using linkages susceptible to biodegradation, such as ester, amide, peptide, carbonate, urea, anhydride, orthoester, phosphazine and phosphoester bonds. The time required for a polymer to degrade can be tailored by selecting appropriate monomers. Differences in crystallinity also alter degradation rates. For relatively crystalline or hydrophobic polymers, actual mass loss may occur by fragmentation or may begin when the oligomeric fragments are small enough to be water soluble. Thus, initial polymer molecular weight and structure will influence the degradation rate.

### FREEZING AND SOLVENT REMOVAL

The fleeces of the invention are prepared by freezing solutions of reactive materials, and then vacuum drying the frozen solutions to produce the lyophilized fleece. Crosslinking can be provided at any point after freezing, including in the frozen state, in the lyophilized state, and during reconstitution with an aqueous solution. Reactive materials may be added after freezing.

The temperature to which the initial solution is frozen may be varied. The temperature of a conventional freezer, about -20 C, is convenient. However, colder or warmer temperatures of freezing may be selected, as long as the frozen solution remains frozen during lyophilization. If non-aqueous solvents are present in the frozen mixture, due attention must be paid to possible effects resulting from differential removal of solvents by lyophilization.

As shown in the examples, it is possible to only partially crosslink the fleece in the frozen or vacuum-dried state, and complete the crosslinking at a later stage. It is also demonstrated that the formed fleece may be shredded, and yet the shredded material can form a coherent mass upon reconstitution. This implies that the material form of the fleece, for at least some purposes, need not be preserved during drying or vacuum drying. Hence, freezing of small droplets, followed by drying in the frozen state, is expected to yield a useful material. Lyophilization may be accelerated by suspension of such particles in a cold dry gas. Solvent removal could also be accelerated by replacement of water with a supercritical fluid, such as supercritical carbon dioxide, especially with an intermediate solvent exchange.

In addition, air or other gas can be incorporated into the matrix to enhance porosity, by the incorporation of bubbles during the freezing step. For example, bubbles of gas can be formed in the macromer solution by any conventional method, and the solution can be frozen immediately. Method for bubble generation include whipping, injection of gas, *in situ* creation of gas (e.g., mixing a carbonate with an acid, or by formation of a urethane bond from an isocyanate, or by action of a metal on a peroxide), and dissolution of gas at high pressure followed by depressurization.

### CROSSLINKING

As described above, the polymer can be any polymer that can be crosslinked in a soluble, frozen or dry state. The type of crosslinking is not critical, and can be covalent, ionic, hydrogen-bonded, or hydrophobic (van der waals) in nature, as long as it can be controlled so that it does not substantially occur until the solution has been at least frozen, and preferably frozen and lyophilized. Preferred for simplicity are polymers that have reactive groups which require activation. Free-radical polymerizable groups, such as ethylenically-unsaturated groups, are particularly simple and easy to use, as will be shown in the examples. As an alternative approach, polymers which will irreversibly aggregate upon freezing may also be useful. In particular, proteins can be useful in such processes. A preferred type of polymer, used in the examples below, is a polymer, having a molecular weight in the range of approximately 2000 to about 1,000,000 Daltons, which has ethylenic groups covalently attached to the polymer.

The broadest range of processes for crosslinking is found in the lyophilized state. In this state, chemically reactive groups can be activated by initiators, by heat, by light, or by the provision of co-reactants. Reagents for crosslinking, including difunctional or multifunctional crosslinkers, can be introduced into the macromer solution, particularly if dissolved in solvents which do not materially swell the lyophilized fleece. Reactive agents can also be applied as a spray, either in their liquid state if applicable, or in a gas or solvent. Ionically crosslinkable polymers can be treated with solutions containing the appropriate ions, once in the fleece state.

A particularly simple method of crosslinking is to provide a material in the initial solution which is part of or associated with the fleece after drying. Then it can be activated by simple processes, such as the provision of heat or light, which minimize or obviate post-crosslinking processing. For example, in the example below, succinoyl peroxide is included in the solution which is frozen. Being non-volatile, it adheres to the lyophilized material, and is easily activated by heat to crosslink ethylenically unsaturated groups attached to the polymer.

Crosslinking can also be performed in the frozen state, before vacuum drying. Many materials can be crosslinked by ionizing radiation, for example. Materials which can be free-radical polymerized or crosslinked can be activated and crosslinked by relatively low doses and energies of radiation, and by ultraviolet light. UV, visible and infrared light can be used if photoinitiators, and optionally electron transfer agents, are included in the frozen solution. Some materials, such as proteins which denature on freezing, may not require additional crosslinking, and can be lyophilized or in some cases dried with no additional reaction.

### BIODEGRADABILITY

In many uses it is preferable if the fleece is biodegradable, i.e., spontaneously disintegrating in the body, or in use, into components which are small enough to be metabolized or excreted, or which will disintegrate sufficiently to allow materials to escape from the fleece, particularly from a gel phase in the fleece, under the conditions normally present in a mammalian organism or living tissue.

Typically, the polymers contain bonds linking subunits of the polymers, or linking reactive groups to the polymers, which degrade at a predictable rate in the environment of use, especially in the body. Suitable biodegradable linkages, as noted above, can be hydroxy-substituted aliphatic carboxylic acids, such as lactic acid, glycolic acid, lactide, glycolide, lactones, for example but not limited to caprolactone, dioxanone, and cyclic carbonates. The degradation time can be controlled by the location of hydroxyl substitution (alpha position is fastest), the local hydrophobicity, and the local steric hindrance at the bond. Other suitable labile bonds include but are not limited to anhydrides, orthocarbonates, orthoesters, acetals, phosphazines and phosphoesters, and peptide bonds in amino acids.

The fleece may be entirely biodegradable. It may be made of biodegradable materials having more than one degradation rate. It also may be made of a mixture of biodegradable and non-biodegradable materials, so that the degradable component will dissolve over a certain period leaving a stable structure of material behind. The fleece may also be made without biodegradability, which is preferred when the end use so permits.

### BIOCOMPATIBILITY

Biocompatibility, in the context of the materials and devices of the invention, is the absence of stimulation of a severe, long-lived or escalating biological response to a fleece applied to tissue, and is distinguished from a mild inflammation which typically accompanies surgery or implantation of foreign objects into a living organism. Biocompatibility may be determined by histological examination of the implant site at various times after implantation. One sign of poor biocompatibility can be a severe, chronic, unresolved phagocytic response at the site. Another sign of poor biocompatibility can be necrosis or regression of tissue at the site. In the preferred embodiment, a biocompatible material elicits minimal or no fibrosis or inflammation. This can be achieved preferably through selection of hydrogel composition, and particularly through the use of hydrogel components resulting in degradation of the hydrogel in vivo in less than about three months, preferably less than about two weeks, more preferably within three to ten days. Such rates of degradation may vary depending on the medical application the biocompatible material is to be used.

### ADDITIVES AND EXCIPIENTS

The initial solution, and thus the formed fleece, can further comprise any additives or excipients which would be useful in the final product in its intended use. These include, without limitation, biologically active agents, biologically derived materials, cells, buffers, salts, osmotic strength controlling agents, preservatives, plasticizers, emollients, initiators, polymerization promoters, and polymers not participating in the polymerization reaction which will at least initially be present in the final product. Any of these materials may be encapsulated, immobilized, coated, or otherwise treated to protect them during processing or to control the rate of their release from the fleece. Particulate materials may be ground to an appropriate size, including among others a size having a characteristic dimension conveniently measured in the millimeter, multimicron, micron or submicron size ranges.

Biologically active agents can be any of the wide variety of substances which can influence the physiology or structure of a living organism. In a chemical sense, the principal classes are small organic molecules, inorganic compounds, and polymeric materials, the polymers including at least proteins, polysaccharides, lipids, nucleic acids and synthetic polymers, and copolymers and conjugates of these. These materials may have any function known in the art. Particular functions include antibiotics, growth regulating molecules, structure-inducing materials, hemostatic agents, materials regulating the attachment or detachment of cells from the hydrated fleece antibodies, antigens, transfection vectors and expression vectors and other nucleic acid constructs, anesthetics, and anti-arrhythmic agents.

The fleeces produced have several advantageous properties. A prominent feature is the "stickiness" exhibited by fleeces made from low-concentration macromer solutions. On exposure to moisture, these fleeces adhere strongly to surfaces, including particularly tissue surfaces. Tissues tested include skin, mucous membranes, surfaces of internal organs, and wounds. The degree of stickiness is concentration dependent, and decreases as the macromer solution in the original solution is decreased. However, the fleeces are much stickier than equivalent concentration hydrogels, when hydrogels will form at all at such low concentrations. Because the fleece can be so sticky, it will be useful to provide a non-sticky backing when the fleece must be handled after wetting.

A second advantageous property is the rapidity of hydration and swelling. Lyophilized materials, including lyophilized preparations of the macromers may be slow to rehydrate and redissolve. However, the fleeces hydrate within seconds, when made from low concentrations of macromer. When solvents are used for rehydration, they are preferably substantially or entirely aqueous solutions, as the fleece is intended to be applied to biological tissue.

A third advantageous property is the flexibility and tensile strength obtained from various manufacturing procedures. In particular, tensile strength does not sharply decline as macromer concentration decreases, nor is it prominently a function of macromer molecular weight. It appears that the strength of the fleece may be derived from interactions among domains of concentrated polymer formed between ice crystals. Moreover, significant differences in the flexibility of the dry fleece are found depending on details of procedure as shown below.

### USES FOR THE FLEECE

The fleeces, along or in combination with active agents, living cells or other additives, can be used for any of a variety of medical purposes. The following uses are a non-exhaustive illustration of potential applications for the fleece. A material that is biodegradable and highly biocompatible, such as the material described in the examples below, is envisaged. In some applications the material should attract cells to its surface.
WOUND TREATMENT: The fleece may be used to stop bleeding, preferably in combination with a hemostatic agent such as thrombin. As used herein, a hemostatic material has the property of stopping the flow of blood, which may include stopping the flow of plasma. A hemostat or hemostatic material may work by any of several mechanisms. It may be used as a wound dressing, where its absorptive properties, non-irritating nature, and potential biodegradability are valuable, particularly in deep, large-area , or burn wounds. The wound dressing is optionally reinforced with a backing, and may contain antibiotics, growth factors, or other materials useful in wound healing. As a hemostat or bandage, the fleece may be left in the wound, where it will degrade in a controlled manner. Because the fleece is strongly adherent to moist tissue, it can be used for these functions by simply removing it from a package and applying it to a wound site. The fleece will adhere to mucous membranes, such as buccal membranes, for a significant length of time. As noted above, after about a second in the presence of body fluid, it will adhere to tissue or to itself. It can thus also be used as a self-adhesive bandage, by impregnating a macroporous substrate, such as a fabric, optionally a biodegradable fabric, with a crosslinkable polymer solution, and carrying the composite materials through freezing and lyophilization, and subsequently crosslinking the polymer. (This is illustrated in the Examples.)
ADHESIVE AND BARRIER: Because it adheres to tissue, the fleece can be used to adhere tissue to other tissue, or to adhere devices to tissue. It is also suitable for use, alone or with releasable drugs or polymers (such as hyaluronic acid), for prevention of the formation of tissue adhesions. In this use, the fleece is placed at the site at which development or redevelopment of adhesions is expected. In any application, it may be placed as a macroscopic piece or pieces, or it may be sprayed or otherwise deposited as a dry powder.
DRUG DELIVERY: The fleece is useful in adhering to tissue for the delivery of drugs and other biologically functional materials. The active materials can be incorporated into the fleece when it is manufactured. If the active material is resistant to the processing, then it can be applied to the fleece just before the fleece is applied to tissue, as a solution or powder. It is especially useful for local delivery of drugs.
CELL CULTURE AND TISSUE ENGINEERING: Because the macropores in the fleece are large enough to accommodate mammalian cells, the fleece can be used as a substrate for culturing cells. In particular, if appropriate factors are provided in the fleece or in a culture medium, cells can grow and if applicable differentiate in the fleece. It is thus possible to fabricate the fleece so that it will return to a desired shape when hydrated; impregnate it with or have adhered to it cells in a growth medium; optionally remove unincorporated cells; and cultivate the composite until it is filled with cells to a desired density. This could be used in the repair of cartilage. It could also be used to provide a scaffold for organ replacement, or for providing bulk at a tissue site. Since multiple layers of differing composition can be frozen, one on another, or previously frozen shapes can be coated with polymer solution of different composition, then provision for differential cell growth or differentiation can be made in such a device. In addition, for this or other uses, the fleece can be limited in expansion volume (and thus in shape) by the incorporation of reinforcing materials, such as degradable or biocompatible fibers, during its preparation.

Examples of tissues which can be repaired and/or reconstructed using the fleece material include nervous tissue, skin, vascular tissue, cardiac tissue, pericardial tissue, muscle tissue, ocular tissue, periodontal tissue, connective tissue such as cartilage, tendon, meniscus, and ligament, organ tissue such as kidney tissue, and liver tissue, glandular tissue such as pancreatic tissue, mammary tissue, and adrenal tissue, urological tissue such as bladder tissue and ureter tissue, and digestive tissue such as intestinal tissues.

### MATERIAL for DELIVERY of LIVING CELLS for TISSUE ENGINEERING

The fleece material can be processed to produce particulates by means of shredding or other methods. When wetted with an aqueous solution, the particulates form a slurry. Living cells, such as chondrocytes, cardiomyocytes, or stem cells, such as mesenchymal stem cells, for example, may be added to the slurry material to aid in delivery of the living cells to a defect as a means of tissue engineering for repair of tissues, such as cartilage or cardiac tissue, for example.

### USE OF FLEECE AS A MATRIX FOR CELL INJECTION

The fleece may be placed in a defect, such as in cartilage defect, for example, and held in place with the use of a membrane or sealant or other means. Living cells may then be injected through the membrane or sealant into the fleece layer, which will absorb the living cells and allow the cells to disperse in the fleece layer, effectively delivering and holding living cells in a defect to allow for tissue repair.

The present invention will be further understood by reference to the following non-limiting examples.

**The following materials are used in the examples:**

PEG-based reactive macromers were used in all of the studies. These materials are available from Genzyme Biosurgery, One Kendall Square, Cambridge, MA 02139, under the trademark "FOCALSEAL^{™}". There are four forms: FOCALSEAL^{™}-S, FOCALSEAL^{™}-L, FOCALSEAL^{™}-M, and FOCALSEAL^{™} Primer. All consist of a core of PEG, partially concatenated with monomers which are linked by hydrolyzable (biodegradable) linkages, and capped at each end with a photopolymerizable acrylate group. These differ based on the molecular weight of the core PEG, the number of PEG molecules, and the number and composition of the biodegradable monomers. FOCALSEAL^{™}-S includes PEG with molecular weight 19,400 ± 4000 Daltons; FOCALSEAL^{™}-L and FOCALSEAL^{™}-M include PEG with molecular weight 35,000 ± 5000 Daltons. FOCALSEAL^{™}-S includes trimethylene carbonate ("TMC") monomers in a ratio of at least six or seven TMC molecules to each PEG, typically twelve to thirteen TMC molecules to each PEG, and lactide monomers, typically four lactide molecules to each PEG molecule, with a maximum of five lactide monomers to each PEG. FOCALSEAL^{™}-M is the same as FOCALSEAL^{™}-S with the exception of the molecular weight of the PEG. FOCALSEAL^{™}-L includes TMC molecules in a ratio of less than ten, more typically seven, TMC molecules to each PEG. U.S. Patent No. 6,083,524 describes the synthesis in detail of these materials.

These materials may be polymerized by preparing a solution containing a photoinitiator system. For example, a 10 g aqueous formulation consists of 1 g FOCALSEAL^{™}-S, 54 mg triethanoloamine (TEOA), 80 mg monopotassium phosphate (KPhos) (1.2% by weight or 19 mM), 40 mg vinylcaprolactam (VC) (0.5% by weight), and 0.4 mg of Eosin-Y (10-100 ppm, preferably 30-60 ppm). Surfactant is preferably added, such as PLURONIC^{™} F127, to 0-1% by weight, and t- butylperoxide is then added to a concentration of typically 0.0125% by weight. The polymerization of the material may be facilitated by the addition of a primer solution, such as FOCALSEAL^{™} primer. This primer contains PEG with a molecular weight of approximately 3350 dalton and approximately five molecules of lactate per PEG, ferrous gluconate (Fe-Gluconate), and Eosin-Y.

Other manners of polymerization may be used. For example, polymerization may be initiated by chemical or thermal free-radical polymerization, redox reactions, cationic polymerization, and chemical reaction of active groups (such as isocyanates, for example.). Certain specific manners of polymerization are described in the following examples.

### EXAMPLE 1. Preparation of a Fleece Comprising Thermally-Activated Polymerization

The following fleeces were prepared:
**1A**: A solution was prepared containing 5.4% (by weight) of a polymerizable macromer in water. The macromer contained a PEG (polyethylene glycol) backbone, molecular weight about 35,000 Daltons as labeled, partially concatenated with TMC (trimethylene carbonate) linkages. Both ends of the concatenated PEG were extended with TMC and lactide groups, and finally terminated with an acrylic acid ester. The synthesis of such materials is described in U.S. Patent Nos. 6,083,524 and 5,410,016, hereby incorporated by reference. The solution also contained 18.2 mg of succinoyl peroxide (Pfalz&Bauer) in 4.0 g of solution. This solution of 4 g was then poured into a 1.5 x 2 inch plastic weight boat to a depth of about 3 mm and was frozen in a freezer to about -20° C. The frozen solution was placed in a lyophilizer and lyophilized for about 42 hrs to dryness. The temperature in the lyophilizer chamber was then raised to about 50° C for 10 hours. The purpose of this step was to thermally activate the succinoyl peroxide, which is non-volatile, to initiate free radical crosslinking of the acrylate-capped macromers. The resulting matrix was firm but flexible. When placed in water the fleece hydrated well into a gelatinous, opaque gel.
**1B**: A solution was prepared containing 5.0 % macromer solution, and 9.28 mg of succinoyl peroxide totaling 4 g was poured into a 1.5 x 2 inch plastic weigh boat to a depth of about 2.5 - 3 mm and was frozen in a freezer to about -20° C. The frozen solution was placed in a lyophilizer and lyophilized for about 42 hrs to dryness. The temperature in the lyophilizer chamber was then raised to about 50° C for 10 hours. The resulting matrix was more flexible than 1A and very resilient. When placed in water the fleece hydrated well into a gelatinous, slightly opaque gel.
**1C**: A solution was prepared containing 5.1 % macromer and containing 1.33 mg of succinoyl peroxide, totaling 4 g, was poured into a 1.5 x 2 inch plastic weigh boat to a depth of about 3 mm and was frozen in a freezer to about -20° C. The frozen solution was placed in a lyophilizer and lyophilized for about 42 hrs to dryness. The temperature in the lyophilizer chamber was then raised to about 50° C for 10 hours. The resulting matrix was more flexible than 1A and 1B and very resilient. When placed in water the fleece hydrated well into a gelatinous, clear gel.
**1D**: A solution was prepared containing 2.96 % macromer and 4.96 mg of succinoyl peroxide, totaling 4 g, and was poured into a 1.5 x 2 inch plastic weigh boat to a depth of about 3 mm and was frozen in a freezer to about -20° C. The frozen solution was placed in a lyophilizer and lyophilized for about 42 hrs to dryness. The temperature in the lyophilizer chamber was then raised to about 50° C for 10 hours. The resulting matrix was more flexible than 1A, 1B and 1C, and very resilient. When placed in water the fleece hydrated well into a gelatinous, clear gel.

Fleece samples were stored in foil bags (to minimize moisture pickup) at room temperature, or in a refrigerator, or at -20°C. The fleeces had tensile strength sufficient for easy handling. On immersion of a piece (about 1x1 cm) of fleece in about 100 ml of water in a beaker, the fleece immediately became hydrated and sank into the solution. Within less than an hour it had swelled to occupy about 40 to 50 mL of volume. It was too slippery/fragile to lift out of the solution, but maintained integrity as observed by swirling the beaker, and by trapping of air in the gel.

In contrast, a solution of macromer, which was frozen and lyophilized but not crosslinked, dissolved on hydration to form a solution, and was too dilute to crosslink by heating to retain or regain its integrity as a fleece.

### EXAMPLE 2. Multilayer Gels

A stock solution of initiator was prepared by dissolving 0.2063 g benzoyl peroxide in 5.0 g t-butyl alcohol (with warming). A stock solution of polymer with a concentration of 9.77 % containing 123.47 mg benzoyl peroxide and 2.88 g of t-butyl alcohol was prepared. After the addition of the initiator, the stock solution was mixed thoroughly for 2 minutes using a microprocessor (Virtis) at 20,000 - 30,000 rpm resulting in an opaque solution. A 3.75 x 7.5inch metal tray was used as a mold. 32 g of DI water was placed into the mold and allowed to freeze at -20° C. This provides a flat surface for the matrix and a potential means of preventing adherence to the mold. The matrix was fabricated by diluting the macromer stock with DI water to a: 2.9%, b: 4.9%, and c: 6.5%. Starting with 20 g of dilution a, the solution was added to the mold and frozen at -20° C. The process was repeated with 20 g of solution b, 25 g of solution c, and a final 25 g layer of stock solution (9.8% macromer concentration) was added. The pre-frozen, multilayer assembly was lyophilized and heated to 50° C over 10 hours, resulting in a crosslinked fleece. It had similar overall properties to example 1A, 1B, and 1C, but was more flexible.

### EXAMPLE 3. Absorption of Blood Using the Fleece

At the conclusion of an operation performed for other purposes, the kidney of an anesthetized, heparinized rabbit was punctured with a scalpel, producing bleeding. Pieces of the material of Example 2 were pushed into the site of bleeding. They initially absorbed blood, which later passed through the blood-wetted fleece. This demonstrated that the pores in the hydrated material were large enough to allow the passage of red cells. The polymer making up the fleece was designed for biocompatibility, and did not provoke clotting in this experiment. This experiment demonstrates potential suitability of the fleece for cell culture, or for hemostatic uses if a suitable hemostatic material is incorporated or impregnated into the fleece.

### EXAMPLE 4: Tissue Adherence of Fleece

Pieces of fleece of the present invention adhered rapidly and strongly to moist tissue. For example, fleece made as described in Example 2 adhered well to moistened or damp hands and buccal membranes (as well as moist surgical gloves). Adherence was maintained until the fleece dried, or was removed (ca. 1 hr., buccal). With the provision of limited water, swelling was likewise limited. The fleece could be backed with a piece of standard cellophane tape, and removed from a site by pulling on the tape. This demonstrates potential use as a wound dressing. With the use of a biodegradable fleece, the wound dressing would not have to be removed from a healing wound. In such a use, a suitable backing material would preferably also be made from a biodegradable material, such as a thin film of concentrated macromer, or an absorbable gelatin-based material.

### EXAMPLE 5. Multilayer Gels with Hemostatic Surface

A stock solution of initiator was prepared by dissolving 0.2024 g benzoyl peroxide in 5.0 g t- butyl alcohol (with warming). A 45 gram stock solution of polymer containing 4.39 g macromer, 67.17 mg benzoyl peroxide and 1.44 g of t-butyl alcohol) was prepared. After the addition of the initiator, the stock solution was mixed thoroughly for 2 minutes using a microprocessor (Virtis) at 20,000 - 30,000 rpm resulting in an opaque solution. A 5 x 5 cm plastic weight boat was used as a mold. 17 g of DI water was placed into the mold and allowed to freeze at -20° C. The matrix was fabricated by diluting the macromer stock with DI water to solution a: 1.8%, solution b: 3.6%, and solution c: 7.2%. 8 g of stock solution (9.75% macromer concentration) was added to the mold and freezing at-20° C. The process was repeated with 6.7g of solution c, 5.38 g of solution b, and 5.38 g of solution a. The matrix was finished with a 5g layer containing 1000 units of Thrombin. The pre-frozen, multilayer assembly was lyophilized and heated to 50° C for 10 hours. It was removed from the mold in a single piece. It had similar overall properties to the fleeces of example 1A and 1B, but was more flexible.

This fleece was tested during a surgical procedure on an animal, and appeared to have hemostatic properties.

### EXAMPLE 6. Multilayer Gels with Anti-Adhesion Layer

Example 5 was repeated constructing a frozen multi-layer matrix. The matrix was finished with a 5.1g layer of 0.4% Hyaluronic Acid (MW 1,000-2,000 K Daltons, from Genzyme) in Phosphate Buffer (PBS). The pre-frozen, multilayer assembly was lyophilized and heated to 50° C for 10 hours. It had similar overall physical properties to the fleeces of examples 1A and 1B and 1C.

### EXAMPLE 7 Incorporation of a Support into the Fleece.

A strip of woven material made of the degradable polymer polyglycolide, (medium weight, Davis&Geck) was impregnated with a thin layer of 5% monomer, and was then placed on top of a 30 g frozen layer of a 5% aqueous solution of macromer. The macromer contained a PEG (polyethylene glycol) backbone, molecular weight about 20,000 Daltons as labeled, partially concatenated with TMC (trimethylene carbonate) linkages, and was extended with TMC and lactide groups, and finally terminated with an acrylic acid ester. The solution contained 5.0 mg of benzoyl peroxide per 30 mL of solution. The composite was lyophilized and crosslinked using conditions discussed in previous examples. The resulting material was flexible and had excellent tensile properties. Like the unsupported fleece, it adhered strongly to moist surfaces, including moist skin. This material may be used as a bandage, alone or impregnated with therapeutic materials.

### EXAMPLE 8. Photocured Fleece.

A 2 gram solution was prepared which contained 10% by weight of the macromer of Example 7 ("20KTLA"), and 4 mg vinylcaprolactone, 0.054g triethanolamine, 0.08g potassium phosphate, and 40 ppm Eosin Y. The solution was frozen in a -20° C freezer. It was illuminated to induce photopolymerization of the macromers in the frozen state, using blue green light (450 - 550 nm, Xenon source) at about 100 mW per square cm., for 40 seconds. The crosslinked material was then lyophilized, leaving a fleece with properties similar to Examples 1A and 1B (which were crosslinked after lyophilization).

### EXAMPLE 9. Photocured Fleece.

A 2 gram solution was prepared which contained 200 mg by weight of the macromer of Example 1 ("35KTLA"), and 2.5 mg vinylcaprolactone, 0.027g triethanolamine neutralized to pH 7.0 with H3PO4, and 20 ppm Eosin Y. The solution was frozen in the -20° C freezer. It was illuminated to induce photopolymerization of the macromers in the frozen state, using blue green light (450 - 550 nm, Xenon source) at about 100 mW per square cm., for 40 seconds. The crosslinked material was then lyophilized, leaving a fleece with properties similar to Examples 1A and 1B (which were crosslinked after lyophilization).

### EXAMPLE 10. Photocured Fleece.

A 2 gram solution was prepared which contained 258 mg by weight of the macromer of Example 1 ("35KTLA"). The solution contained 1.31 mg vinylcaprolactone, 0.143 g triethanolamine neutralized to pH 7.0 with H3P04 and ppm 15 ppm Eosin Y. The solution was frozen in a -20° C freezer. It was illuminated to induce photopolymerization of the macromers in the frozen state, using blue green light (450 - 550 nm, Xenon source) at about 100 mW per square cm., for 80 seconds. The crosslinked material was then lyophilized, leaving a fleece with properties similar to Example 1C and 1D (which were crosslinked after lyophilization).

### EXAMPLE 11. Slurry Preparation from Photocured Fleece.

A 3.12% (by weight) solution was prepared by diluting with a buffer a stock solution of polymerizable FOCALSEAL-S macromer (10% by weight) as described above. A 10.0 g formulation of the 3.12% solution contained: 3.12 g of the stock solution, 332.0 mg N-Vinyl-Caprolactam, 6.55 g buffer (containing 0.035 g Triethanolamine, 0.052 g Monobasic-Potassium Phosphate, 1.25 µL t-butylhydroxide (70% in water) and 0.26 mg Eosin Y). Gels were prepared using 0.6g -0.8 g of this formulation and illuminated to induce photopolymerization of the macromers at room temperature using blue green light (450 - 550 nm, Xenon source) at about 100 mW per square cm., for 80 seconds. The gels were placed into 200 mL of DI water at room temperature and allowed to soak for approximately 60 minutes. Water was decanted from gels. Fresh 200 mL DI water was added again and gels allowed to soak for an additional 35 minutes. Gels were collected using a coarse sintered glass funnel then transferred gels into a 250 mL tall beaker containing approximately 100 mL DI water. Gels were shredded for 60 seconds at 30,000 rpm using a Virtis Microprocessor with ultra fine blade (# 255193). Gel particles were collected using a medium size sintered glass filter. Approximately 30 mL of Gel particles /water suspension was subsequently lyophilized.

Initially the construct was evaluated for suitability as a slurry using 1-2 mg of polymer and wetting it with only 1-2 drops of DI water. A total of 169 mg construct with a sponge-like consistency was obtained. The dry, fluffy construct was then proportioned into small quantities of approximately 9 mg -11 mg using PS petri dishes, double (tyvek) bagged and sterilized using EtO for evaluation in a goat model.

### EXAMPLE 12. Slurry Preparation from Photocured Fleece.

A 5.0% (by weight) solution was prepared by diluting with a buffer the stock solution described in Example 11. A 10.00 g formulation of the 5.0% solution contained: 5.01 g of the stock solution (10% concentration), 280.0 mg N-Vinyl-Caprolactam, 4.71 g buffer (containing 0.025 g Triethanolamine, 0.037 g Monobasic-Potassium Phosphate, 0.089 µL t-Butylhydroxide (70% in water) and 0.19 mg Eosin Y). Gels were prepared using 0.5g -0.8 g of this formulation and illuminated for 80 seconds to induce photopolymerization of the macromers at room temperature using blue green light (450 - 550 nm, Xenon source) at about 100 mW per square cm. The gels were placed into 200 mL of DI water at room temperature and allowed to soak for approximately 30 minutes. Water was decanted from gels. Fresh 200 mL DI water was added again and gels allowed to soak for an additional 45 minutes. Gels were collected using a coarse sintered glass funnel then transferred gels into a 250 mL tall beaker containing approximately 100 mL DI water. Gels were shredded for 90 seconds at 30,000 rpm using a Virtis Microprocessor with ultra fine blade (# 255193). When larger gel fractions were observed shredding was continued for an additional 60 seconds. The gel particles were collected using a medium size sintered glass filter. The gel particles/water suspension was subsequently lyophilized. A total of 155 mg somewhat granular but fluffy material was obtained.

The construct was evaluated for suitability as a slurry using 1-2 mg of polymer and wetting it with only 1-2 drops of DI water. Construct showed coarser particles compared to the slurry prepared in Example 11.

### EXAMPLE 13. Slurry Preparation from Fleece containing Hyaluronic acid (HA)

A 3.0% (by weight) solution was prepared by diluting with a buffer the stock solution described in Example 11 and Hyaluronic acid (HA, MW 1,500kDa).

A 20.045 g formulation of the 3.0% solution contained: 6.012 g of the stock solution (10% concentration), 659.8 mg N-Vinyl-Caprolactam, 1.4387 g of buffer (containing: 0.07769 g Triethanolamine, 0.1151 g Monobasic-Potassium Phosphate, 2.73 µL t-Butylhydroxide (70% in water) and 0.58 mg Eosin Y), 8.0128 g Sepracoat (0.4% HA) and 3.9215 g water. Gels were prepared in a teflon mold: 1.5 cm in diameter and 0.4 mm - 0.8 mm deep; then illuminated for 80 seconds to induce photopolymerization of the macromers at room temperature using blue green light (450 - 550 nm, Xenon source) at about 100 mW per square cm. The gels were placed into 500 mL of DI water at room temperature after illumination to prevent dehydration. The gels were washed with 3x500mL of DI water over a two hour time period. Water was decanted from gels, then transferred into a 250 mL tall beaker containing approximately 150 mL DI water. The gels were shredded for 60 seconds at 30,000 rpm using a Virtis Microprocessor with ultra fine blade (# 255193). The shredded material was kept at room temperature for one hour then transferred into 2 x 50 mL conical tubes and centrifuged for 14 minutes at 2500 rpm. Water was removed from the gel pellet. The washing /centrifugation cycle was repeated. The gel particles/water suspension was subsequently lyophilized. A total of 568 mg dry particulate material was obtained.

### EXAMPLE 14. Slurry Preparation from Photocured Fleece containing Acrylate-PEG-RGD

A 2.76% (by weight) solution was prepared by diluting with a buffer the stock solution described in Example 11 and addition of acrylated PEG-RGD peptide (RGD peptide contains arginine-glycine-aspartic acid sequence). A 21.762 g formulation contained: 5.9964 g of the stock solution (10% concentration), 683.8 mg N-Vinyl-Caprolactam, 1.4101 g buffer (containing 0.0756 g Triethanolamine, 0.112 g Monobasic-Potassium Phosphate, 2.7 µL t-Butylhydroxide (70% in water) and 0.56 mg Eosin Y), 13.336 g water, 0.2509 acrylated PEG-RGD (Acrylated PEG-RGD (prepared by coupling Acrylated-PEG-NHS [Shearwater Polymers] with RGD peptide [Sigma Chemicals]). Gels were prepared in a teflon mold: 1.5 cm in diameter and 0.4 mm - 0.8 mm deep; then illuminated for 80 seconds to induce photopolymerization of the macromers at room temperature using blue green light (450 - 550 nm, Xenon source) at about 100 mW per square cm. The gels were placed into 500 mL of DI water at room temperature after illumination to prevent dehydration. The gel batch was washed with 3x500mL of DI water over a two hour time period. Water was decanted from gels, then transferred into a 250 mL tall beaker containing approximately 150 mL DI water. Shredded gels for 60 seconds at 30,000 rpm using a Virtis Microprocessor with ultra fine blade (# 255193). The shredded material was kept at room temperature for one hour then transferred into 2 x 50 mL conical tubes and centrifuged for 14 minutes at 2500 rpm. Water was removed from the gel pellets. The washing /centrifugation cycle was repeated. The gel particles/water suspension was subsequently lyophilized. A total of 564 mg dry slurry material was obtained.

### EXAMPLE 15. Slurry Preparation from Photocured Fleece containing TGF-β

A 2.79% (by weight) solution was prepared by diluting with a buffer the stock solution described in Example 11 and addition of TGF-β.

A 21.762 g formulation of the 2.79% solution contained: 6.033 g of the stock solution (10% concentration), 660.2 mg N-Vinyl-Caprolactam, 1.4154 g buffer (containing 0.0764 g Triethanolamine, 0.113 g Monobasic-Potassium Phosphate, 2.7 µL t-Butylhydroxide (70% in water) and 0.. 57 mg Eosin Y, 13.310 g water, 0.1685 g TGF-β. Gels were prepared in a Teflon mold: 1.5 cm in diameter and 0.4 mm - 0.8 mm deep; then illuminated for 80 seconds to induce photopolymerization of the macromers at room temperature using blue green light (450 - 550 nm, Xenon source) at about 100 mW per square cm. The gels were placed into 500 mL of DI water at room temperature after illumination to prevent dehydration. The gels batch was washed with 3x500mL of DI water over a two hour time period. Water was decanted from gels, then transferred into a 250 mL beaker containing approximately 150 mL DI water. Gels were shredded for 60 seconds at 30,000 rpm using a Virtis Microprocessor with ultra fine blade (# 255193). The shredded material was kept at room temperature for one hour then transferred into 2 x 50 mL conical tubes and centrifuged for 14 minutes at 2500 rpm. Water was removed from the gel pellet. The washing /centrifugation cycle was repeated. The gel particles/water suspension was subsequently lyophilized. A total of 564 mg dry particulate material was obtained.

### EXAMPLE 16. Shredded Fleece Preparation using Redox Curing

Two separate 5.0 g solutions were prepared which contained 0.748 g (in DI water) of the macromer of Example 1 ("35KTLA"). To solution # 1 was added 0.0989 g of Ferrous gluconate. To solution # 2 was added 0.00978 g of t-butyl peroxide. Gels were prepared by utilizing a dual syringe system (1.0 mL each) for static mixing, which was fitted with a pre-molded modified delivery tip containing a screw type mixing thread. A gel formed when the contents of the syringes were mixed. Gels so prepared were placed into about 150 mL of DI water and cut manually into smaller pieces. Using a Virtis Microprocessor and spinning blade # 307686 the gels were cut into smaller fragments over a 5 minute period. This was then changed to blade # 225185, a micro fine adapter, for 5 to 10 minutes, and then changed to an ultra fine blade # 25,5193 for 10 minutes. The fragments were collected using a filter with a 100,000 MW cut off membrane. The gel fragments were freeze dried. The resulting material was cotton like with a weak structure.

### EXAMPLE 17. Fleece Preparation using Redox Curing

Example 16 was followed in gel preparation and processing of gels, and fragmentation, except 0.0986 g Phosphate Buffer pH 7.5 was added to redox solution # 2 prior to mixing the two components. The processed and subsequently freeze-dried matrix dried to a thinner film with gauze like properties.

### EXAMPLE 18. Fleece Preparation using gel fragments

A fleece was fabricated using gel fragments from Example 17 then placed in a freezer at -20° C. Gel fragments from Example 16 were used as a second layer, frozen and then topped with gel fragments from Example 17. The frozen matrix was lyophilized and resulted in a single matrix with flexible properties.

### EXAMPLE 19. Absorption of Blood Using the Fleece

At the conclusion of an operation performed for other purposes, the kidney of an anesthetized, heparinized rabbit was punctured with a scalpel, producing bleeding. A 3 x 0.8 cm x approximately 2-4 mm thick patch of the material of Example 18 was pressed into the site of bleeding. The patch absorbed the blood without any break through on one occasion. In a second attempt the thickness of the patch was doubled in order to stop break through of blood. This demonstrated that the pores in the hydrated material were large enough to allow the passage of red cells and that there is a potential for use in hemostasis with this formulation.

### EXAMPLE 20: Use of Fleece for Support of Living Cells

A pellet of cultured cartilage cells containing about 2.5 million cells was resuspended in about 5 ml of growth medium. A disc of fleece of Example 8, about 0.6 cm in diameter, was placed in the bottom of a Petri dish, and the cell suspension was added slowly onto the fleece. Within less than a minute, the fleece had expanded and imbibed the entire solution. No segregation of the cells to the surface was visually observable, and it is believed that the cells adhered to the pores and crevices of the expanded fleece.

### EXAMPLE 21: Preparation of Fleece with Air Bubbles in the Macromer Gel

A formula essentially identical to that of Example 8 was frozen before polymerization, and further had air incorporated by a micronization (high shear mixing) procedure. The resulting fleece was fluffy and had a fibrous structure, and rehydrated rapidly (less than 1 minute.) Adhesion to tissue was lower than Example 1, presumably because of the higher macromer concentration.

### EXAMPLE 22: Viability of Living Cells in Slurry Preparation

10 mg of fleece particulate material made by the process described in Example 11 is placed on a millipore filter, which is placed in a 24 well plate (the filter holds the gel together).

The fleece particulate material is pre-wetted with 23 ml/mg of media, (Dulbecco's Modified Eagle's Medium (DMEM)), or about 230 µl/10 mg of material, in order to prewet the material prior to adding living cells. The mixture of fleece particulate material and media is allowed to stand for about 30-45 minutes. This allows the material to form a gel of a proper consistency of a slurry. Pre-wetting the fleece particulate material before introducing cells is preferable to avoid cell death through dessication.

To add the living chondrocyte cells to the slurry, the cells are trypsinized and pelletized then resuspended in a very small volume of media, i.e. 50 µl and gently dispersed through the slurry. The medium can either be Dulbecco's Modified Eagle's Medium (DMEM) supplemented with 10% (v/v) fetal bovine serum or another defined medium. About 0.4 ml of media was placed around the outside of the filter to supply nutrient to the cells.

Place the plate in a 37°C humidified incubator for a couple of hours. Add about 0.4 mls of media to the gel. Add the media very gently so as not to disperse the gel.

### Viability Assay of slurry with living cells, as described above.

| | |
|---|---|
| Slurry Preparation at 24 hrs | 98% Cell Viability |
| Slurry Preparation at 72 hrs | 84% Cell Viability |

### EXAMPLE 23. Cartilage Repair Using Slurry Preparation

A test was conducted to determine the feasibility of delivering chondrocytes in a slurry to a focal full-thickness chondral defect in a goat's knee.

### Materials and Methods:

### Cell Preparation:

Articular cartilage was harvested from the non-weight-bearing portion of the lateral trochlear ridge of the distal femur of a goat. The harvested cartilage was rinsed with DMEM, and placed in 0.25% protease for approximately 1 hour at 37°C/5% CO₂. After one hour, the protease is removed, the cartilage is washed 2x with Ham's F12 medium, and 0.1% collagenase is added to the tissue overnight at 37° C/5%CO₂. The collagenase is quenched with 10% Fetal Bovine Serum (FBS), and the sample spun for 5 minutes @ 1000 rpms. The cell pellet is resuspended in complete medium (10% FBS/DMEM). Cells are counted and plated into T75 flasks with 20 mls of complete medium.

Cells are expanded in culture until 90% confluency, trypsinased, counted, pelleted and resuspended in DME/10% FBS. Cells are frozen at 5x10^5-1x10^6/amp. depending on the total cell count. The amps are placed O/N in N2 interface and placed in the Jacuzzi the next day. Cells were stored until time of implantation.

At the time of implantation, the cells are released from the culture plates with trypsin-EDTA, counted, and suspended in serum-free medium (DME) at a concentration of 30 million cells per 100 µl. Cell suspension was diluted with 100 µl of serum-free medium in the operating room for each animal, and an aliquot of cell suspension was mixed with the fleece particulates to form a slurry. The fleece particulates were prepared as described in Example 11.

### Implantation surgery:

Six-mm diameter full-thickness chondral defects were created in the center of the lateral facet of the patella of each knee of each goat. A primer solution containing ferrous gluconate, as described above, was applied to the defect surface (cartilage walls and bottom surface) using a brush to work the material into the surface interstices. Each defect was filled to 1/3 of its total depth with the slurry material containing living cells. Only a small percentage of total prepared material was used. The slurry was pressed into the corners of defect at the cartilage-bone interface, and pressed lightly into the bottom of the defect to form a smooth surface. An aliquot of the cell composite was evaluated for cell viability. The slurry was covered with FocalSeal-S sealant (refer to prior art), filling the defect completely, and the sealant was photopolymerized using a Focal, Inc.-supplied light source and light wand, delivering visible wavelength in the blue-green region. Two timed cycles for a total of 80 seconds of photopolymerization was used. Each joint was closed and the animal recovered after the second implantation was completed.

### Necropsy and histologic evaluation:

One animal was sacrificed at 3 days and one at four weeks after implantation. Joints were examined and synovial fluid, synovial membrane, the patellar defect, trochlea, meniscus, and fat pad were harvested from each joint. In the animal sacrificed at 3 days, the repair tissue within each defect was removed for frozen sectioning. In the animal sacrificed at 4 weeks, the defect was fixed in 10% neutral buffered formalin, embedded in plastic, serial sectioned and stained with Toluidine blue or hematoxylin and eosin stain. All remaining tissues from both animals were fixed in 10% neutral buffered formalin, embedded in paraffin, cut in 5 µm sections, and stained with hematoxylin and eosin. Synovial fluid from the four-week time point joints was centrifuged, decanted, and the supernatant frozen at-80°C, and synovial smears were made from fluid from the right stifle joint.

### Results:

### Surgery:

An aliquot from one preparation of the cell composite from each animal was tested for viability at the time of implantation. The assay was run approximately 1-2 hours after the cells were suspended in the material. Cells were viable in both preparations tested; however, the viability in one preparation was below 70%, the acceptable viability for Autologous Chondrocyte Implantation (ACI) cell suspension. The low cell viability of the implants may be due to the omission of the pre-wetting step as described in Example 22.

The cell composite was easy to implant, and the entire implantation took only a few minutes, compared to 30-45 minutes for ACI. The slurry material conformed well to the irregularities of the cartilage and bone surfaces of the defect.

### Necropsy at Three Days:

The synovial fluid was slightly red-tinged with normal viscosity in both joints. The joint capsule was reddened. Overall the joint appeared normal for three days post-arthrotomy.

The defect in the left patella was grossly filled to 20% of the defect depth with soft, translucent material, some of which had the appearance of hydrogel in the dependent portion. There was a significant amount of sloping of the adjacent cartilage walls into the defect, and the fibrillated edges from the communicating Grade 4 lesion present at surgery were swollen into the defect, accounting for some of the tissue fill within the defect. Histology of the patellar defect (post removal of the repair tissue) showed moderate numbers of neutrophils infiltrated into an otherwise acellular material that appeared eosinophilic and fibrillar with small, clear spaces separating fibrils. No obvious viable chondrocytes were present in the small amount of material left in the defect, as expected due to omission of the pre-wetting of the fleece particulates prior to adding the living cells. No bacteria or other etiologic agent was present in the section to account for the neutrophilic inflammation. The walls of the adjacent cartilage varied in the degree of degeneration from mild to marked through the serial sections and from one side to the other.

The defect in the right patella was grossly filled to 60-70% of defect depth, and the implant appeared intact. The edges of the defect were described as clean with no fissures. Histologic analysis was not performed on the defect post-removal of the implant.

Removal of the gel material appeared to remove most of the repair tissue from each defect. The samples that were collected were the polymerized hydrogel surface layer that contained a film-like residue on the basal margin. Histology on the removed repair tissue in both defects showed individual to small clusters of cells was fairly evenly scattered through the FOCALSEAL material and present along the basal margin. The cells appeared to be imbedded in little to no endogenous matrix. Cell viability of the tissue in the left defect was 15.6% and 18.9% in the right defect, again the omission of pre-wetting the fleece particulates may have caused the living cells to dessicate.

### I. NECROPSY AT FOUR WEEKS

The defect in the left patellofemoral joint was grossly filled to 50% of its depth with white, granular tissue, which was primarily connected to the defect edges. Histologic evaluation revealed fibroblastic cells throughout the repair tissue, which appeared to contain a large amount of hydrogel. The defect in the right patellofemoral joint was grossly filled to 80% of its depth with smooth, off-white tissue, with an uneven surface and covered with a yellow film. Histologic evaluation showed neutrophils and macrophages in the repair tissue. No etiology for the inflammation was evident.

In summary, the slurry system was delivered and retained in the defect at 3 day and 4 week time points. The implant was at a minimum partially retained in all four defects. One defect at 3 days was only 20% filled grossly, suggesting some implant loss; however, the tissue present contained some viable cells. This defect had soft, irregular edges and communicated with a Grade 4 lesion. Previous studies in our laboratory have shown difficulties retaining periosteal grafts in tissue with this level of degeneration, so even partial retention of the implant is positive.

Viable cells were demonstrated within the repair/composite implant tissue at three days post-implantation. Although the percentage of viable cells was low, the slurry particulates were not pre-wetted and the cells were likely subjected to dessication, and the cell concentration may not have been optimal for cell survival and proliferation.

Delivery of the cell composite required less time than for cell delivery using ACI, and had the additional advantage of less risk of cell loss than ACI. Although chondrogenic tissue was not produced as a result of delivery with this system, the slurry conditions had not been optimized, and model used has not been validated as a model of cartilage repair, and may not have resulted in repair using ACI. Nevertheless, the present system resulted in delivery of viable cells, with complete implant retention in three of four defects and partial retention in one defect with significantly compromised edges. Early signs of repair tissue was evident in both defects at the four-week time point. The composite could be delivered rapidly without invading the cartilage adjacent to the defect.

The invention is not limited by the embodiments described above which are presented as examples only but can be modified in various ways within the scope of protection defined by the appended patent claims.

Thus, while there have been shown and described fundamental novel features of the invention as applied to a preferred embodiment thereof, it will be understood that various omissions and substitutions and changes in the form and details of the devices illustrated, and in their operation, may be made by those skilled in the art without departing from the spirit of the invention. For example, it is expressly intended that all combinations of those elements and/or method steps, which perform substantially the same function in substantially the same way to achieve the same results, are within the scope of the invention. Moreover, it should be recognized that structures and/or elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto.

## Claims

1. A process for making a biocompatible biodegradable fleece, the process comprising:
a. providing a solution comprising a crosslinkable synthetic macromer, the synthetic macromer comprising a polymeric hydrophilic region surrounded by two or more regions each comprising one or more moieties forming a biodegradable region and a crosslinkable moiety;
b. freezing the solution in a desired shape;
c. vacuum-drying the solution; and
d. crosslinking the crosslinkable macromer to produce the fleece.

2. The process of claim 1 wherein the vacuum-drying step is performed before or after the crosslinking step.

3. The process of claim 1 or 2 wherein the macromer solution further comprises at least one of a polymerization-causing material and a biologically active agent.

4. The process of any of claims 1 to 3 wherein the biologically active agent is selected from the group consisting of antibiotics, growth regulating molecules, hemostatic agents, antibodies, antigens, transfection vectors, expression vectors, anesthetics, and antiarrhythmic agents.

5. The process of any of claims 1 to 4, wherein the crosslinking is performed by the use of at least one of ionizing radiation, non-ionizing radiation, heat, addition of initiators, and addition of crosslinking chemicals or ions.

6. The process of any of claims 1 to 5, wherein the crosslinking is performed by a free radical polymerization reaction.

7. The process of any of claims 1 to 6 further comprising a rinsing of the crosslinked macromer.

8. The process of any of claims 1 to 7 further comprising the step of shredding the crosslinked macromer after rinsing.

9. The process of any of claims 1 to 8 further comprising the step of shredding the crosslinked macromer to form fleece particulates.

10. The process of any of claims 1 to 9 further comprising the step of shredding the crosslinked macromer after at least one of the freezing step and the vacuum-drying step.

11. The process of any of claims 1 to 10 wherein a supporting material is incorporated into the fleece.

12. The process of any of claims 1 to 11 where the incorporation of the supporting material occurs during the freezing step.

13. The process of any of claims 1 to 9, further comprising the wetting of the fleece particulates with an aqueous solution.

14. The process of any of claims 1 to 13 further comprising the adding of at least one of a cell, a polymerization-causing material, and a biologically active agent to the wetted fleece particulates.

15. The process of any of claims 1 to 14, wherein bubbles are incorporated into the solution before the freezing step.

16. A biocompatible biodegradable fleece produced by the process of claim 1, having both macroporosity and, when hydrated, microporosity.

17. The biocompatible biodegradable fleece according to claim 16, wherein the fleece is macroporous having a pore size in the range of microns to millimeters.

18. The fleece of claim 16 or 17, further comprised of at least one of a cell, a polymerization-causing material and a biologically active agent.

19. The fleece of any of claims 16 to 18, comprising a diacrylated polyethylene oxide comprising biodegradable linkages selected from the group consisting of monomers and oligomers of carbonates and hydroxyacids.

20. The fleece of any of claims 16 to 19, further comprised of at least one of a cell, a polymerization-causing material, and a biologically active agent.

21. The fleece of any of claims 16 to 20 which is in the form of fleece particulates.

22. The fleece of any of claims 16 to 21, wherein the fleece has at least two regions of differing composition.

23. The process of any of claims 1 to 14, wherein the crosslinkable macromer is water soluble.

24. A slurry comprising the biocompatible fleece particulates of claim 21 and an aqueous solution.

25. The slurry of claim 24, wherein the aqueous solution comprises at least one of a cell, a polymerization-causing material, and a biologically active agent.

26. The slurry of claims 24 or 25, which comprises cells selected from the group consisting of chondrocytes, cardiomyocytes, and non-human embryonic stem cells.

27. Use of the slurry of any of claims 24 to 26 for the preparation of a medicament for treating a wound or tissue defect.

28. Use according to claim 27 wherein the slurry comprises living cells.

29. Use according to claim 27 or 28 wherein the slurry comprises cells selected from the group consisting of chondrocytes, and cardiomyocytes.

30. Use according to any of claims 27 to 29, wherein the non-human embryonic stem cells are mesenchymal stem cells.

31. Use according to any of claims 27 to 30 wherein the tissue defect is a chondral defect, and the living cells are chondrocytes.

32. Use according to any of claims 27 to 31 in combination with a primer solution.

33. Use according to any of claims 27 to 32 in combination with a sealant.

34. Use according to claim 33, wherein the sealant is a biodegradable, polymerizable macromer.

35. Use according to claim 34, wherein the sealant is polymerizable by use of at least one of ionizing radiation, non-ionizing radiation, heat, addition of initiators, and addition of crosslinking chemicals or ions.

36. Use according to any of claims 27 to 35 in combination with a hemostatic agent.

37. Use of the biocompatible biodegradable fleece of claims 16 to 23 for the preparation of a medicament for preventing tissue adhesions in combination with releasable drugs or polymers.

## Patentansprüche

1. Verfahren zum Herstellen eines biokompatiblen, biologisch abbaubaren Vlieses, wobei das Verfahren umfasst:
a) Bereitstellen einer Lösung, die ein vernetzbares synthetisches Makromer umfasst, wobei das synthetische Makromer einen polymeren, hydrophilen Bereich aufweist, der von zwei oder mehr Bereichen umgeben ist, welche jeweils eine oder mehrere Einheiten aufweisen, die einen biologisch abbaubaren Bereich und eine vernetzbare Einheit bilden;
b) Einfrieren der Lösung in einer gewünschten Form;
c) Vakuumtrocknen der Lösung und
d) Vernetzen des vernetzbaren Makromers, um das Vlies herzustellen.

2. Verfahren nach Anspruch 1, wobei der Vakuumtrocknungsschritt vor oder nach dem Vernetzungsschritt durchgeführt wird.

3. Verfahren nach Anspruch 1 oder 2, wobei die Makromerlösung ferner zumindest ein Polymerisations-verursachendes Material und/oder ein biologisch aktives Agens umfasst.

4. Verfahren nach einem der Ansprüche 1 bis 3, wobei das biologisch aktive Agens aus der Gruppe ausgewählt ist, die aus Antibiotika, Wachstums-regulierenden Molekülen, hämostatischen Agenzien, Antikörpern, Antigenen, Transfektionsvektoren, Expressionsvektoren, Anästhetika und antiarrhythmischen Agenzien besteht.

5. Verfahren nach einem der Ansprüche 1 bis 4, wobei die Vernetzung durch Verwendung von zumindest ionisierender Strahlung, nichtionisierender Strahlung, Wärme, Zugabe von Initiatoren, und/oder Zugabe von vernetzenden Chemikalien oder Ionen durchgeführt wird.

6. Verfahren nach einem der Ansprüche 1 bis 5, wobei die Vernetzung mittels einer freiradikalischen Polymerisationsreaktion durchgeführt wird.

7. Verfahren nach einem der Ansprüche 1 bis 6, das ferner ein Spülen des vernetzten Makromers umfasst.

8. Verfahren nach einem der Ansprüche 1 bis 7, das ferner den Schritt des Zerkleinerns des vernetzten Makromers nach dem Spülen umfasst.

9. Verfahren nach einem der Ansprüche 1 bis 8, das ferner den Schritt des Zerkleinerns des vernetzten Makromers zur Bildung von Vliesteilchen umfasst.

10. Verfahren nach einem der Ansprüche 1 bis 9, das ferner den Schritt des Zerkleinerns des vernetzten Makromers nach zumindest dem Einfrierschritt und/oder dem Vakuumtrocknungsschritt umfasst.

11. Verfahren nach einem der Ansprüche 1 bis 10, bei dem ein Trägermaterial in das Vlies integriert wird.

12. Verfahren nach einem der Ansprüche 1 bis 11, wobei das Integrieren des Trägermaterials während des Einfrierschrittes erfolgt.

13. Verfahren nach einem der Ansprüche 1 bis 9, das ferner das Benetzen der Vliesteilchen mit einer wässrigen Lösung umfasst.

14. Verfahren nach einem der Ansprüche 1 bis 13, das ferner die Zugabe zumindest einer Zelle, eines eine Polymerisation verursachenden Materials und/oder eines biologisch aktiven Agens zu den benetzten Vliesteilchen umfasst.

15. Verfahren nach einem der Ansprüche 1 bis 14, bei dem vor dem Einfrierschritt Blasen in die Lösung eingebracht werden.

16. Biokompatibles, biologisch abbaubares Vlies, das durch das Verfahren nach Anspruch 1 hergestellt wird und sowohl Makroporosität als auch, wenn es hydratisiert ist, Mikroporosität aufweist.

17. Biokompatibles, biologisch abbaubares Vlies nach Anspruch 16, wobei das Vlies makroporös ist und eine Porengröße im Mikrometer- bis Millimeter- Bereich aufweist.

18. Vlies nach Anspruch 16 oder 17, das ferner zumindest aus einer Zelle, einem eine Polymerisation verursachenden Material und/oder einem biologisch aktiven Agens besteht.

19. Vlies nach einem der Ansprüche 16 bis 18, das ein diacryliertes Polyethylenoxid mit biologisch abbaubaren Bindungen aufweist, die aus der Gruppe ausgewählt sind, welche aus Monomeren und Oligomeren von Karbonaten und Hydroxysäuren besteht.

20. Vlies nach einem der Ansprüche 16 bis 19, das ferner zumindest aus einer Zelle, einem eine Polymerisation verursachenden Material und/oder einem biologisch aktiven Agens besteht.

21. Vlies nach einem der Ansprüche 16 bis 20, das in Form von Vliesteilchen vorliegt.

22. Vlies nach einem der Ansprüche 16 bis 21, wobei das Vlies zumindest zwei Bereiche unterschiedlicher Zusammensetzung aufweist.

23. Verfahren nach einem der Ansprüche 1 bis 14, wobei das vernetzbare Makromer wasserlöslich ist.

24. Aufschlämmung, welche die biokompatiblen Vliesteilchen nach Anspruch 21 und eine wässrige Lösung umfasst.

25. Aufschlämmung nach Anspruch 24, wobei die wässrige Lösung zumindest eine Zelle, ein Polymerisations-verursachendes Material und/oder ein biologisch aktives Agens umfasst.

26. Aufschlämmung nach den Ansprüchen 24 oder 25, welche Zellen umfasst, die aus der Gruppe ausgewählt sind, welche aus Chondrozyten, Kardiomyozyten und nicht-menschlichen Embryonenstammzellen besteht.

27. Verwendung der Aufschlämmung nach einem der Ansprüche 24 bis 26 zur Herstellung eines Medikamentes zur Behandlung einer Wunde oder eines Gewebedefektes.

28. Verwendung nach Anspruch 27, wobei die Aufschlämmung lebende Zellen umfasst.

29. Verwendung gemäß Anspruch 27 oder 28, wobei die Aufschlämmung Zellen umfasst, die aus der Gruppe ausgewählt sind, welche aus Chondrozyten und Kardiomyozyten besteht.

30. Verwendung nach einem der Ansprüche 27 bis 29, wobei die nicht-menschlichen Embryonenstammzellen mesenchymale Stammzellen sind.

31. Verwendung nach einem der Ansprüche 27 bis 30, wobei der Gewebedefekt ein chondraler Defekt ist und die lebenden Zellen Chondrozyten sind.

32. Verwendung nach einem der Ansprüche 27 bis 31 in Kombination mit einer Primerlösung.

33. Verwendung nach einem der Ansprüche 27 bis 32 in Kombination mit einem Dichtmittel.

34. Verwendung nach Anspruch 33, wobei das Dichtmittel ein biologisch abbaubares, polymerisierbares Makromer ist.

35. Verwendung nach Anspruch 34, wobei das Dichtmittel polymerisierbar ist durch Verwendung zumindest von ionisierender Strahlung, nichtionisierender Strahlung, Wärme, Zugabe von Initiatoren und/oder Zugabe von vernetzenden Chemikalien oder Ionen.

36. Verwendung nach einem der Ansprüche 27 bis 35 in Kombination mit einem hämostatischen Agens.

37. Verwendung des biokompatiblen, biologisch abbaubaren Vlieses der Ansprüche 16 bis 23 zur Herstellung eines Medikamentes zum Verhindern von Gewebeanhaftungen in Kombination mit freisetzbaren Wirkstoffen oder Polymeren.

## Revendications

1. Processus de réalisation d'une toison biocompatible et biodégradable, ce processus comprenant :
a. réalisation d'une solution comprenant un macromère synthétique réticulable, le macromère synthétique comprenant une région polymère hydrophile entourée de deux régions ou plus, chacune comprenant un ou plusieurs groupes fonctionnels formant une région biodégradable et un groupe fonctionnel réticulable ;
b. congélation de la solution afin d'obtenir une forme souhaitée ;
c. séchage sous vide de la solution ; et
d. réticulation du macromère réticulable afin de produire la toison.

2. Processus de la revendication 1 dans lequel l'étape de séchage sous vide est effectuée avant ou après l'étape de réticulation.

3. Processus de la revendication 1 ou 2 dans lequel la solution de macromère comprend, en outre, au moins un des éléments suivants : un matériau provoquant la polymérisation et un agent biologiquement actif.

4. Processus selon l'une des revendications 1 à 3 dans lequel l'agent biologiquement actif est sélectionné dans le groupe constitué d'antibiotiques, de molécules de régulation de la croissance, d'agents hémostatiques, d'anticorps, d'antigènes, de vecteurs de transfection, de vecteurs d'expression, d'anesthésiques et d'agents antiaryhtmiques.

5. Processus selon l'une des revendications 1 à 4 dans lequel la réticulation est effectuée grâce à l'utilisation d'au moins une radiation ionisante, une radiation non ionisante, de la chaleur, l'ajout d'initiateurs et l'ajout de produits chimiques ou d'ions de réticulation.

6. Processus selon l'une des revendications 1 à 5 dans lequel la réticulation est effectuée grâce à une réaction de polymérisation par radicaux libres.

7. Processus selon l'une des revendications 1 à 6 comprenant en outre un rinçage du macromère réticulé.

8. Processus selon l'une des revendications 1 à 7 comprenant en outre l'étape de déchiquetage du macromère réticulé après le rinçage.

9. Processus selon l'une des revendications 1 à 8 comprenant en outre l'étape de déchiquetage du macromère réticulé pour former des particules de toison.

10. Processus selon l'une des revendications 1 à 9 comprenant en outre l'étape de déchiquetage du macromère réticulé après au moins une des étapes suivantes : l'étape de congélation et l'étape de séchage sous vide.

11. Processus selon l'une des revendications 1 à 10 dans lequel un matériau de support est incorporé dans la toison.

12. Processus selon l'une des revendications 1 à 11 dans lequel l'incorporation du matériau de support est effectuée pendant l'étape de congélation.

13. Processus selon l'une des revendications 1 à 9 comprenant en outre l'humidification des particules de toison avec une solution aqueuse.

14. Processus selon l'une des revendications 1 à 13 comprenant en outre l'ajout d'au moins un des éléments suivants : une cellule, un matériau provoquant la polymérisation et un agent biologiquement actif, aux particules de toison humidifiées.

15. Processus selon l'une des revendications 1 à 14 dans lequel des bulles sont incorporées dans la solution avant l'étape de congélation.

16. Toison biocompatible biodégradable produite à l'aide du processus de la revendication 1, ayant une macroporosité et, lorsqu'elle est hydratée, une microporosité.

17. Toison biocompatible biodégradable selon la revendication 16 dans laquelle la toison est macroporeuse avec une taille de pores de l'ordre de quelques microns à quelques millimètres.

18. Toison selon la revendication 16 ou 17, constituée en outre d'au moins un des éléments suivants : une cellule, un matériau provoquant la réticulation et un agent biologiquement actif.

19. Toison selon l'une des revendications 16 à 18 comprenant un oxyde de polyéthylène diacrylaté comprenant des liaisons biodégradables sélectionnées dans le groupe constitué de monomères et d'oligomères de carbonates et d'hydroxyacides.

20. Toison selon l'une des revendications 16 à 19 constituée en outre d'au moins un des éléments suivants : une cellule, un matériau provoquant la polymérisation et un agent biologiquement actif.

21. Toison selon l'une des revendications 16 à 20 qui se présente sous la forme de particules de toison.

22. Toison selon l'une des revendications 16 à 21 dans laquelle la toison comprend au moins deux régions de compositions différentes.

23. Processus selon l'une des revendications 1 à 14 dans lequel le macromère réticulable est soluble dans l'eau.

24. Suspension comprenant les particules de toison biocompatible de la revendication 21 et une solution aqueuse.

25. Suspension selon la revendication 24 dans laquelle la solution aqueuse comprend au moins un des éléments suivants : une cellule, un matériau provoquant la réticulation et un agent biologiquement actif.

26. Suspension selon les revendications 24 ou 25 qui comprend des cellules sélectionnées dans le groupe constitué de chondrocytes, de cardiomyocytes et de cellules souches embryonnaires non humaines.

27. Utilisation de la suspension selon l'une des revendications 24 à 26 pour la préparation d'un médicament permettant de traiter une blessure ou un défaut des tissus.

28. Utilisation selon la revendication 27 dans laquelle la suspension comprend des cellules vivantes.

29. Utilisation selon la revendication 27 ou 28 dans laquelle la suspension comprend des cellules sélectionnées dans le groupe constitué de chondrocytes et de cardiomyocytes.

30. Utilisation selon l'une des revendications 27 à 29 dans laquelle les cellules souches embryonnaires non humaines sont des cellules souches mésenchymales.

31. Utilisation selon l'une des revendications 27 à 30 dans laquelle le défaut des tissus est un défaut chondral et les cellules vivantes sont des chondrocytes.

32. Utilisation selon l'une des revendications 27 à 31 en combinaison avec une solution amorce.

33. Utilisation selon l'une des revendications 27 à 32 en combinaison avec un enduit.

34. Utilisation selon la revendication 33 dans laquelle l'enduit est un macromère biodégradable polymérisable. -

35. Utilisation selon la revendication 34 dans laquelle l'enduit est polymérisable à l'aide d'au moins un des éléments suivants : un rayonnement ionisant, un rayonnement non ionisant, de la chaleur, l'ajout d'initiateurs et l'ajout de produits chimiques ou d'ions de réticulation.

36. Utilisation selon l'une des revendications 27 à 35 en combinaison avec un agent hémostatique.

37. Utilisation de la toison biocompatible biodégradable selon les revendications 16 à 23 pour la préparation d'un médicament permettant d'empêcher des adhérences de tissus en combinaison avec des médicaments ou des polymères libérables.
